# EUROPÄISCHE PATENTSCHRIFT

(11) **EP 1 997 904 B1**
(45) Veröffentlichungstag und Bekanntmachung des Hinweises auf die Patenterteilung: **27.04.2011**
(21) Anmeldenummer: 07010624.0
(22) Anmeldetag: 29.05.2007
(51) Int. Cl.: C12Q 1/68

(54) **Verfahren zur fälschungssicheren Identifizierung einer auf einem Gegenstand vorgesehenen Markierung**
Method for forgery-proof identification of a marking on an object
Procédé destiné à l'identification infalsifiable d'une marque prévue sur un objet

(43) Veröffentlichungstag der Anmeldung: 03.12.2008
(73) Patentinhaber: Secutech International Pte. Ltd., Singapore 069443 (SG)
(72) Erfinder: Bochmann, Hans-Günther, 90556 Cadolzburg/Wachendorf (DE); Kosak, Hans, 53115 Bonn (DE)
(74) Vertreter: UEXKÜLL & STOLBERG

(56) Entgegenhaltungen:
- WO-A-01/51652
- WO-A-95/30749
- WO-A-99/34984
- WO-A-2006/053435
- US-A- 5 139 812

## Beschreibung

Die vorliegende Erfindung betrifft ein Verfahren zur fälschungssicheren Identifizierung einer auf einem Gegenstand vorgesehenen Markierung, insbesondere von Dokumenten und wertvollen Objekten, mit Hilfe von Nukleinsäuren und einem Vor-Ort-Nachweis der Markierung auf einem markierten Gegenstand. Das Verfahren dient damit der Echtheitsprüfung oder Authentifizierung des Gegenstandes. Unter dem Begriff Gegenstand hierin oft zusammenfassend für Dokumente und anderweitige Objekte verwendet wird.

Durch den weltweit steigenden Dokumenten- und Güterverkehr steigt der Bedarf an sicheren Identifikationsmerkmalen, die in der Lage sind, die Dokumente oder Güter eindeutig zu identifizieren und somit vor Fälschungen zu schützen. Die Markierung der Dokumente oder Güter mit DNA (Desoxyribonukleinsäure) ist eine Möglichkeit für eine eindeutige Markierung. Der Vorteil der Verwendung von DNA ist zum einen die hohe Fälschungssicherheit und zum anderen die Möglichkeit durch Verwendung unterschiedlicher Sequenzen eine Vielzahl von unterschiedlichen Markierungen herzustellen.

Markierungen mit DNA als fälschungssichere Authentifizierungsmerkmale sind seit 1987 bekannt. In dem in WO 1 987 006 383 A1 beschriebenen Verfahren wird eine Markierungs-DNA bekannter Sequenz auf oder in das zu authentifizierende Objekt gegeben und die Markierungs-DNA zur Authentifizierung des Objekts nachgewiesen. Die Markierungs-DNA wird in diesem Verfahren zur Identifizierung isoliert und mit molekular-biologischen Methoden wie z.B. PCR oder Hybridisierung in einem Labor nachgewiesen. Nachteilig ist, dass die Identifikation der Markierungs-DNA in diesem Verfahren labortechnische Geräte wie z.B. PCR-Geräte und technisch hochgeschultes Personal benötigt und zeitaufwändig ist.

Verfahren zur Markierung von wertvollen Gütern oder zu schützenden Dokumenten mit Nukleinsäuren und zum Vor-Ort-Nachweis der Markierung auf einem markierten Gegenstand oder Dokument sind aus den Dokumenten DE 10 000 629 B4 und DE 10 105 339 B4 bekannt. Unter Vor-Ort-Nachweis werden hierin Testverfahren verstanden, die am normalen Empfangsort der Gegenstände mit einfachen Mitteln, ohne Labortechnik, von eingewiesenem Personal einfach vorgenommen werden können.

DE 10 000 629 B4 offenbart ein Verfahren, bei dem zur Markierung auf einen Gegenstand eine spezifische Markierungs-DNAwund benachbart eine unspezifische Kontroll-DNA fixiert werden. Zur Identifizierung der Markierung wird eine zu der Markierungs-DNA komplementäre Nachweis-DNA mit der Markierung auf dem Gegenstand in Kontakt gebracht. Dabei kommt es zu einer spezifischen Hybridisierung zwischen der Markierungs- und der Nachweis-DNA. Die Hybridisierung bewirkt ein verändertes optisches Verhalten der Nachweis-DNA. Die Änderung des optischen Verhaltens kann vor Ort nachgewiesen werden und dient zur Identifizierung des markierten Gegenstands.

DE 10 105 339 B4 beschreibt ein Etikett zur fälschungssicheren Markierung von Gegenständen. Das Etikett besteht aus zwei Feldern, die durch einen kommunizierenden Porenraum verbunden sind. Auf dem ersten Feld befindet sich eine spezifische Markierungs-DNA und auf dem zweiten Feld eine unspezifische Kontroll-DNA. Zur Authentifizierung des Etiketts wird eine Flüssigkeit mit einer Nachweis-DNA, die komplementär zu der Markierungs-DNA ist, auf den Porenraum aufgetragen. Die Flüssigkeit verteilt sich durch Kapillarkräfte auf die beiden Felder des Etiketts. Im Feld mit der Markierungs-DNA kommt es zu einer spezifischen Hybridisierung. Dagegen kommt es im Feld mit der unspezifischen Kontroll-DNA zu keiner Hybridisierung. Die Hybridisierung kann anhand einer veränderten Fluoreszenz nachgewiesen werden. Bei dem Nachweis wird das zweite Feld mit der unspezifischen Kontroll-DNA als Referenzfeld genutzt, um den Intensität der Fluoreszenz zu erhalten, der sich ohne spezifische Hybridisierung einstellt.

Nachteilig an den oben aufgeführten Verfahren ist, dass die Markierung offen sichtbar auf dem Gegenstand aufgetragen ist. Dadurch ist einem möglichen Fälscher die Eigenschaft des Dokuments oder Gegenstands, markiert zu sein, bekannt.

Aufgabe der vorliegenden Erfindung ist es, ein verbessertes Verfahren zur fälschungssicheren Identifizierung von markierten Gegenständen mit Hilfe von Nukleinsäuren bereitzustellen.

Die Aufgabe wird gemäß der vorliegenden Erfindung durch die Merkmale des Anspruchs 1 gelöst. Die abhängigen Unteransprüche erfassen weitere vorteilhafte Ausführungsformen des erfindungsgemäßen Verfahrens.

Das erfindungsgemäße Verfahren zur Identifizierung einer auf einem Gegenstand vorgesehenen Markierung zur Fälschungssicherung umfasst die folgenden Schritte:
a) Bereitstellen eines zu identifizierenden Gegenstands, der zumindest auf einem ersten Markierungsfeld zur Markierung des Gegenstands oberflächlich eine erste Markierungssubstanz enthält, die eine erste Markierungsnukleinsäure mindestens teilweise bekannter Sequenz aufweist,
b) Aufbringen einer Schablone in einer definierten Orientierung auf einen vorgegebenen Oberflächenbereich des Gegenstands, wobei die Lage des ersten Markierungsfeldes durch die Schablone angezeigt wird,
c) Bereitstellen einer ersten Nachweissubstanz, die eine erste Nachweisnukleinsäure aufweist, die zumindest teilweise komplementär zu der ersten Markierungsnukleinsäure ist,
d) Aufbringen der ersten Nachweissubstanz auf das erste Markierungsfeld, unter Bedingungen bei denen die erste Nachweisnukleinsäure spezifisch an die erste Markierungsnukleinsäure bindet und wobei durch die Bindung eine optische Eigenschaft der ersten Nachweisnukleinsäure verändert wird,
e) Messung der optischen Eigenschaft auf dem ersten Markierungsfeld und
f) Identifizieren der Markierung anhand der optischen Eigenschaft auf dem ersten Markierungsfeld.

Mit dem erfindungsgemäßen Verfahren ist es möglich, die Markierung in einer für das menschliche Auge nicht sichtbaren Weise auszuführen, so dass das Vorhandensein der Markierung an dem Gegenstand nicht ohne weiteres erkennbar ist.

Unter einer Schablone im Sinne der vorliegenden Erfindung ist eine Vorrichtung zu verstehen, die auf dem markierten Gegenstand aufgebracht werden kann und die im aufgebrachten Zustand zumindest die Lage eines Markierungsfelds auf dem Gegenstand anzeigt, das auf eine mögliche Markierung geprüft werden soll. Die Schablone ist vorteilhaft so ausgebildet, das sie zumindest eine Aussparung ausweist, durch welche die Nachweissubstanz mit der Nachweisnukleinsäure auf die Markierung aufgetragen werden kann.

Für das Aufbringen der Schablone auf den Gegenstand muss die Orientierung der Schablone und der Oberflächenbereich des Gegenstands, der von der Schablone abgedeckt werden soll, eindeutig definiert und vorgegeben sein. Dies kann zum Beispiel bei Dokumenten dadurch erzielt werden, dass die Schablone die gleiche Größe und Geometrie wie das Dokument hat und sowohl das Dokument als auch die Schablone eine eindeutig ausgezeichnete Orientierungsrichtung haben. Der Oberflächenbereich und die Orientierung können auch durch sichtbare Markierungen bestimmt sein, die nur dem wissenden Benutzer als Orientierungshilfe zur Identifizierung des Gegenstands auffallen. Dazu kann z.B. auf dem Gegenstand ein Rahmen vorgezeichnet sein, der mit der Kontur der Schablone oder Teilen von ihr übereinstimmt und in den die Schablone passend eingelegt wird; der Rahmen muss natürlich nicht durchgängig vorgezeichnet sein, es können auch nur einzelne Punkte der Kontur der Schablone vorgesehen sein, z.B. die Eckpunkte einer mehreckigen flachen Schablone. In noch sichereren Ausführungsformen soll möglichst vermieden werden, dass ein Fälscher mit bloßem Auge überhaupt die Tatsache erkennen kann, dass ein Gegenstand markiert ist; dazu können die Orientierungshilfen auch in Form von optisch aktiven Substanzen wie Fluorophoren oder IR-aktiven Stoffen aufgetragen sein. Diese optisch aktiven Substanzen sind für das Auge ohne Hilfsmittel unsichtbar. Mit Hilfe eines Fluoreszenz-Handscanners oder anderen Hilfsmitteln können die Orientierungshilfen dann sichtbar gemacht werden und zum Auflegen der Schablone dienen.

Bei dreidimensionalen Gegenständen kann auch eine dreidimensionale Geometrie der Schablone vorgesehen sein, die komplementär zu einem bestimmten Oberflächenbereich des Gegenstands ausgebildet ist, so dass ein passgenaues Anlegen nur in einer bestimmten Orientierung an den Gegenstand gelingt.

Das erfinderische Verfahren erlaubt es, die Markierung für das Auge versteckt aufzubringen. Dadurch ist es einem potentiellen Fälscher nicht bewusst, dass der Gegenstand markiert ist. Ohne im Besitz der Schablone zu sein, ist der potentielle Fälscher somit nicht in der Lage, die Markierung zu erkennen. Dies erhöht die Sicherheit der Markierung.

Weiterhin nachteilig an den in der Einleitung aufgeführten Verfahren des Standes der Technik ist es, dass nur eine Ja/Nein-Information und keine Kodierung von komplexer Information möglich ist. Ein weiterer Nachteil ist die Notwendigkeit eines Kontrollfeldes zur Bestimmung des unspezifischen Hintergrunds auf der Markierung. Die vorliegende Erfindung soll es auch erlauben, diese Nachteile in bevorzugten Ausführungsformen zu überwinden.

Vorteilhafterweise ermöglicht es die Verwendung einer Schablone, das Referenzfeld, das zur Bestimmung des unspezifischen Hintergrunds verwendet wird, von dem markierten Gegenstand weg auf die Schablone zu übertragen. Dadurch vereinfacht sich der Aufbau der Markierung, da auf dem markierten Gegenstand kein Referenzfeld vorhanden sein muss.

Weiterhin ist es bei einer Verwendung der Schablone möglich, auch die optischen Merkmale zur Steuerung des Scanprozesses auf der Schablone anzuordnen. Dies reduziert den Aufwand der Markierung und die Fläche der Markierung auf dem Gegenstand. Darüber hinaus ermöglicht diese Ausgestaltung, die Nukleinsäuremarkierung auf einem farblich unruhigen Untergrund anzubringen, da Störungen aufgrund von Farbeigenschaften des Untergrunds auf dem Objekt, die den Scanprozess zur Identifizierung der Markierung stören könnten, durch die Schablone verdeckt werden können. Es ist z.B. nach einer Ausgestaltung der Erfindung möglich, die Markierungsnukleinsäure in einzelnen Buchstaben auf einem Dokument zu verstecken. Nach dem Aufbringen der Schablone und dem Zufügen der Nachweisnukleinsäure wird beim Scanprozess mit dem Handscanner nur der Bereich der Markierungsnukleinsäure auf dem markierten Objekt erfasst. Die Hilfslinien zur Steuerung des Scanprozesses und das Referenzfeld können sich auf der Schablone befinden und sind somit unabhängig von einer möglichen Störung durch die Beschaffenheit des markierten Objekts.

Weiterhin kann eine Schablone verwendet werden, die eine Mehrzahl verschiedener Aussparungen aufweist. Die verschiedenen Aussparungen können z.B. nummeriert sein. Die Aussparungen können in einer definierten Weise mit einer Mehrzahl von Feldern mit Markierungsnukleinsäure (Markierungsfeldern) und Feldern ohne Markierungsnukleinsäure (Referenzfeldern) korrespondieren. Bei einem Nachweis der Markierungsnukleinsäure durch Zufügen von Nachweisnukleinsäure durch die Aussparungen kann dadurch ein komplexer Kode gebildet und entschlüsselt werden. Beispielsweise kann eine binäre Zahl kodiert werden, die darauf beruht, dass durch die Schablone anhand der Nummerierung eine definierte Reihenfolge von Ja-Antworten (Nachweisnukleinsäure auf einem Feld mit Markierungsnukleinsäure) und Nein-Antworten (Nachweisnukleinsäure auf einem Feld ohne Markierungsnukleinsäure) auf dem markierten Objekt abgefragt werden. Die Ja- und Nein-Antworten bilden dann einen binären Kode. In dieser Ausformung erlaubt die Verwendung der Schablone eine komplexe Informationskodierung auf dem Objekt.

Die Schablone ist vorteilhafterweise so ausgestaltet, dass sie in einer eindeutigen Orientierung auf das markierte Objekt, z.B. ein Blatt eines Dokuments, aufgelegt werden kann. Um dies zu erreichen, können auf der Schablone und/oder dem Objekt sichtbare Merkmale in Form von optischen Markierungen, z.B. Strichen, aufgebracht sein. Die Schablone kann auch eine dreidimensionale Struktur besitzen, die mit der Form des markierten Gegenstands korrespondiert, so dass eine eindeutige Orientierung der Schablone auf dem Gegenstand gewährleistet ist. In dieser Ausformung ist es möglich, Markierungen auf Objekten, die nicht eben sind, anzuzeigen und einer Identifizierung durch Zufügen von Nachweisnukleinsäure zugänglich zu machen.

In dem Verfahren enthält das erste Markierungsfeld vorzugsweise ein synthetisches Oligonukleotid bekannter Sequenz, das vorzugsweise in einem Überschuss unspezifischer DNA versteckt ist. Vorzugsweise enthält ein zweites Feld nur die unspezifische oder keine DNA (Referenzfeld). Die Felder sind meist drucktechnisch z.B. mittels Tintenstrahldruckverfahren auf das Objekt aufgebracht oder auf einem Etikett gedruckt, das mit dem Objekt verbunden wird. Zwischen den Feldern sind vorzugsweise drucktechnisch aufgebrachte Farbbalken, z.B. Linien oder Striche, vorgesehen, welche die Erkennung der Position des Markierungs- und Referenzfeldes beim Nachweisprozess z.B. mit einem Fluoreszenz-Handscanner erleichtern.

Zur Identifizierung wird eine Nachweissubstanz, meistens in Form einer Flüssigkeit, auf die Markierung aufgebracht. Diese Nachweissubstanz enthält die Nachweisnukleinsäure, die zumindest partiell zu der Markierungsnukleinsäure komplementär ist. Die verwendete Nachweisnukleinsäure ändert bei einer Hybridisierung seine optische Eigenschaft, z.B. die Intensität der Fluoreszenz. Bei dem Verfahren wird das veränderte Fluoreszenzsignal vorzugsweise mittels eines Fluoreszenz-Handscanners nachgewiesen. Zum Nachweis der Hybridisierung der Nachweisnukleinsäure mit der Markierungsnukleinsäure werden dabei die Felder mit der Markierungsnukleinsäure (Markierungsfeld) und der unspezifischen Substanz oder Nukleinsäure (Referenzfeld) mit dem Fluoreszenz-Handscanner überstrichen. Die Fluoreszenzintensitäten beider Felder werden bestimmt. Die Markierung wird dann als authentisch erkannt, wenn das Verhältnis der Intensitäten der Fluoreszenz zwischen dem Markierungs- und Referenzfeld über einem definierten Wert liegt. Zur besseren Erkennung der Bereiche des Markierungs- und Referenzfeldes sind im Umgebungsbereich der Felder optische Merkmale, z.B. farbige Striche, Linien oder Balken, aufgebracht, die der Fluoreszenz-Handscanner erkennt. Mit Hilfe dieser Balken kann der Messprozess des Scanners gesteuert werden, so dass eine automatische Signalverarbeitung und Authentifizierung erfolgen kann.

In dem Verfahren liegt die Nachweisnukleinsäure vorzugsweise in der Form von sogenannten Molecular Beacon vor. Molecular Beacon sind synthetische Nukleinsäuresequenzen mit einer besonderen Struktur. Molecular Beacon weisen eine einzelsträngigen Schleife und eine endständige Rückfaltung auf, so dass das 5'- und das 3'-Ende der Nukleinsäure miteinander hybridisieren. Am 5'- und 3'-Ende sind ein Fluorophor und ein sogenannter Quencher immobilisiert. In der rückgefalteten Struktur der Molecular Beacon sind Fluorophor und Quencher in unmittelbarer Nachbarschaft. Durch diese enge räumliche Beziehung kommt es bei einer Anregung des Fluorophors mit Licht zu einem strahlungslosen Übertragung der vom Fluorophor absorbierten Energie auf den Quencher. Dadurch wird die Fluoreszenz unterdrückt. Bei einer Hybridisierung eines Molecular Beacon mit einer komplementären Sequenz (Markierungsnukleinsäure) wird die Rückfaltung der Molecular Beacon aufgehoben. Dadurch vergrößert sich der Abstand zwischen Fluorophor und Quencher. Bei einer Anregung des Fluorophors mit Licht, z.B. aus dem Fluoreszenz-Handscanner, kommt es im hybridisierten Zustand des Molecular Beacon zur Emission eines Fluoreszenzsignals.

Weiterhin ist die Erfindung auf ein Kit zur Ausführung eines der beanspruchten Verfahren gerichtet, nämlich Kit zum Identifizieren eines markierten Gegenstands, der eine oberflächlich aufgebrachten Markierungsubstanz an einem vorgegebenen Ort enthält, die eine erste Markierungsnukleinsäure mindestens teilweise bekannter Sequenz aufweist, mit
a) einer Schablone, wobei die Schablone auf den Gegenstand in definierter Weise so aufbringbar ist, dass sie nach Aufbringung den Ort der Markierung anzeigt, und
b) einer Flüssigkeit, die eine zur ersten Markierungsnukleinsäure zumindest teilweise komplementären Nachweisnukleinsäure enthält und die in einem Mittel zur volumendefinierten Abgabe (z.B. einer Pipette) der Flüssigkeit enthalten ist.

Im Folgenden wird das erfindungsgemäße Verfahren anhand eines Ausführungsbeispiels in den Figuren 1 bis 5 erläutert.
Figur 1 zeigt eine Markierung und eine Anwendung der Schablone.
Figur 2 zeigt eine Identifizierung der Markierung aus Figur 1.
Figur 3 und 4 zeigen den gleichen Prozess wie Figuren 1 und 2 mit Referenzfeld auf der Schablone und ohne Referenzfeld auf dem markierten Dokumentenblatt.
Figur 5 zeigt ein Dokumentenblatt mit einer Mehrzahl von Markierungen und deren Identifizierung.

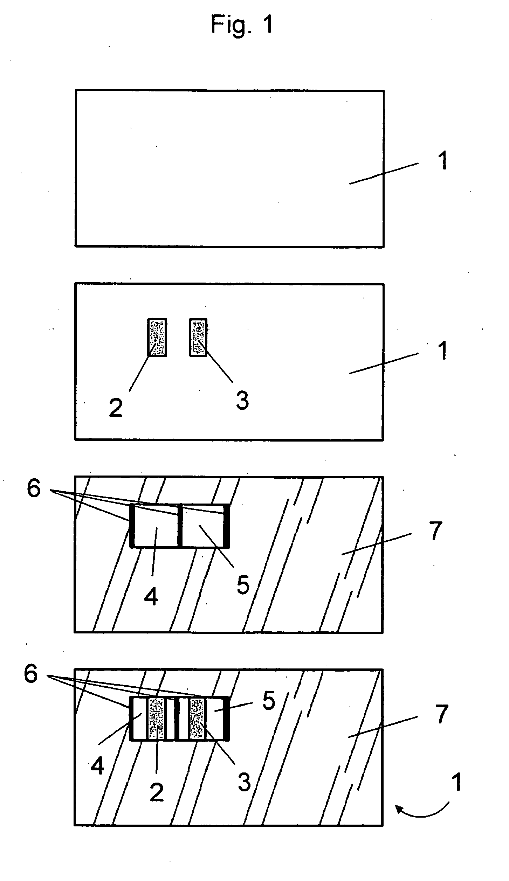

In Figur 1 ist oben ein Gegenstand in Form eines Dokumentenblatts 1 (leeres Rechteck) gezeigt. Auf das Dokumentenblatt 1 wird zur fälschungssicheren Markierung auf einem ersten Markierungsfeld 2 Markierungsnukleinsäure und auf einem zweiten Referenzfeld 3 unspezifische Nukleinsäure aufgebracht (zweites Rechteck von oben). Das dritte Rechteck von oben zeigt schraffiert die Schablone 7, die in diesem Fall die gleiche Geometrie wie das Dokumentenblatt 1 hat. Die Schablone 7 besitzt komplementär zu dem ersten Markierungsfeld 2 und dem zweiten Referenzfeld 3 Aussparungen 4 und 5, die durch dunkle Balken 6 (Hilfslinien für den Scanprozess) markiert sind.

Im vierten Rechteck ist die Schablone 7 gezeigt, nachdem sie maximal bedeckend auf das Dokumentenblatt 1 aufgelegt wurde. Durch die Aussparungen 4 und 5 der Schablone 7 sind das erste Markierungsfeld 2 bzw. das zweite Referenzfeld 3 auf dem Dokumentenblatt 1 sichtbar.

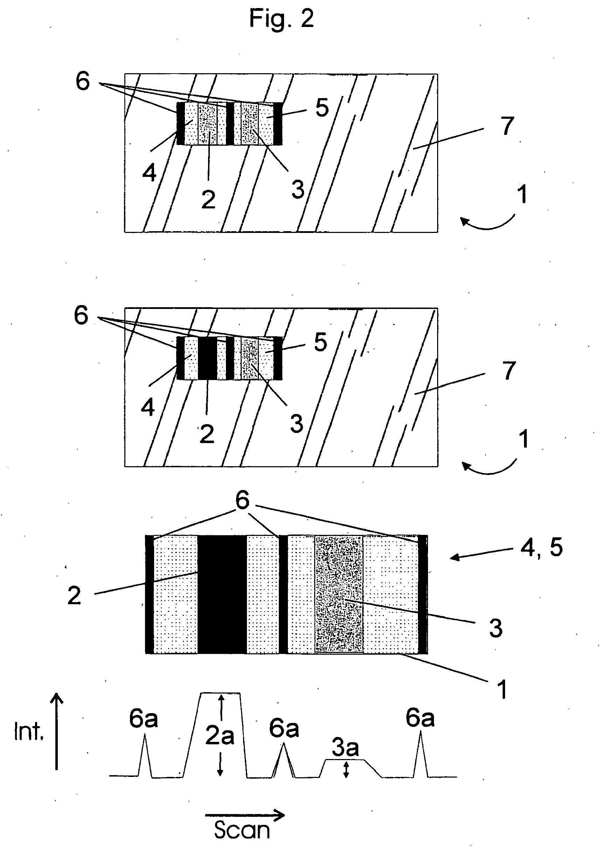

Ganz oben in Figur 2 ist das Dokumentenblatt 1 mit aufgelegter Schablone 7 zu sehen, wobei die Nachweissubstanz auf das erste Markierungsfeld 2 und das zweite Referenzfeld 3 aufgetragen wurde. Darunter ist zu sehen, dass nach wenigen Minuten das erste Markierungsfeld 3 mit der Markierungsnukleinsäure im Vergleich zum zweiten Referenzfeld 3 dunkler geworden ist. Damit soll die Änderung der Intensität der Fluoreszenz aufgrund der spezifischen Hybridisierung der Markierungs- mit der Nachweisnukleinsäure dargestellt werden.

Darunter sind in der Figur 2 die Aussparungen 4 und 5 vergößert dargestellt und die Auswertung des Scanprozesses darunter zeigt, wie sich die Verteilung der Intensität bei einem Überstreichen der Markierung (hier von links nach rechts) mit einem Fluoreszenz-Handscanner darstellt. Die Hilfslinien 6 werden von der Software des Handscanners als Start- und Stoppsignale 6a erkannt (Spitzen in der Auswertung). Der dazwischen liegende Bereich zeigt die Fluoreszenzintensitäten 2a, 3a auf dem Markierungsfeld 2 bzw. Referenzfeld 3 an (oben abgeflachte Erhebungen). Die Software vergleicht die Fluoreszenzintensitäten 2a, 3a des Markierungsfeldes 2 und des Referenzfeldes 3 und bewertet die Markierung ab einem definierten Intensitätsverhältnis 2a/3a zwischen dem Markierungsfeld 2 und dem Referenzfeld 3 als authentisch.

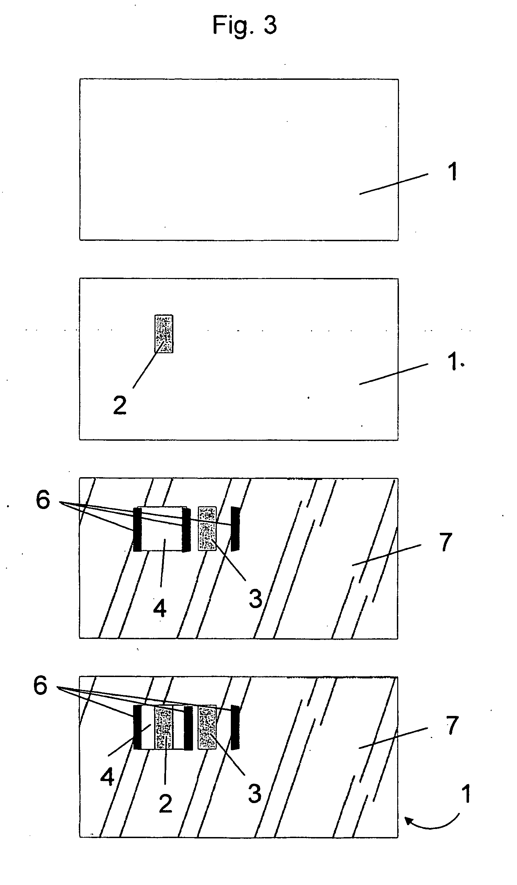

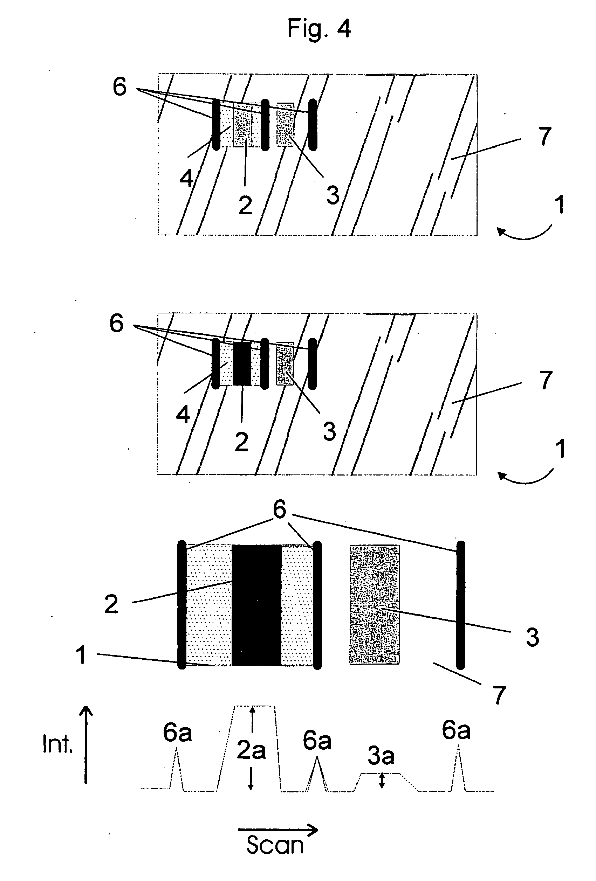

Die Figuren 3 und 4 zeigen ein ähnliches Verfahren wie in den Figuren 1 und 2, wobei das Referenzfeld 3 auf der Schablone aufgebracht ist und nicht auf dem markierten Dokumentenblatt 1. Daher weist die Schablone 7 nur eine Aussparung 4 auf und der Umgebungsbereich des Referenzfeldes 3 auf der Schablone 7 entspricht in etwa der Aussparung 5 in den Figuren 1 und 2.

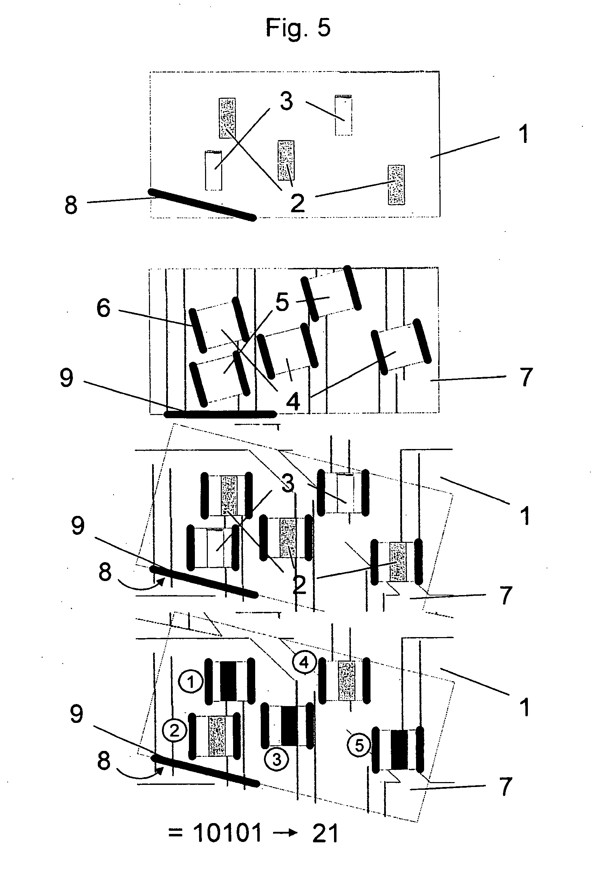

In Figur 5 stellen auf dem Dokumentenblatt 1 die grau gefüllten Balken erste Markierungsfelder 2 mit Markierungsnukleinsäure und die schraffierten Balken Referenzfelder 3 ohne Markierungsnukleinsäure dar. Weiterhin ist auf dem Dokumentenblatt 1 links unten eine Orientierungslinie 8 gezeigt, die zur Positionierung der Schablone dient. Darunter ist eine Schablone 7 dargestellt, die eine Mehrzahl von Aussparungen 4, 5 besitzt. Weiterhin zeigt die Schablone 7 unten links eine Orientierungslinie 9, die zur Positionierung der Schablone dient. Darunter ist gezeigt, wie die Schablone 7 so auf dem Dokumentenblatt 1 orientiert wird, dass die Orientierungslinien 8, 9 auf dem Dokumentenblatt bzw. der Schablone in Deckung sind. Dadurch kommen die Aussparungen 4, 5 der Schablone mit den Markierungs- bzw. Referenzfeldern 2, 3 auf dem Dokumentenblatt zur Deckung.

Ganz unten ist die Situation nach dem Aufbringen der Nachweissubstanz auf die Markierungs- und Referenzfelder 2, 3 gezeigt. Die Markierungsfelder 2 mit Markierungsnukleinsäure sind dunkler dargestellt, wodurch eine gegenüber den Referenzfeldern 3 ohne Markierungsnukleinsäure erhöhte Fluoreszenzintensität während eines Scanprozesses mit einem Fluoreszenz-Handscanner verdeutlicht werden soll.

Soweit wie dargestellt die Schablone 7 für die Aussparungen 4, 5 eine Nummerierung aufweist (Ziffern 1-5), lässt sich aus der Reihenfolge der positiven und negativen Antworten der Markierungs- und Referenzfelder 2, 3 z.B. ein binärer Kode ableiten. Wenn die Nummerierung der Aussparungen 4, 5 zum Beispiel der Bitposition einer binären Zahlendarstellung entspricht, ist in diesem Fall eine Bitfolge 10101 gezeigt, die wiederum der Zahl 21 im Dezimalsystem entspricht.

## Patentansprüche

1. Verfahren zur fälschungssicheren Identifizierung einer auf einem Gegenstand (1) vorgesehenen Markierung mit den folgenden Schritten:
a) Bereitstellen eines zu identifizierenden Gegenstands (1), der zumindest in einem ersten Markierungsfeld, (2) zur Markierung des Gegenstands oberflächlich eine erste Markierungssubstanz enthält, die eine erste Markierungsnukleinsäure mindestens teilweise bekannter Sequenz aufweist,
b) Aufbringen einer Schablone (7) in einer definierten Orientierung auf einen vorgegebenen Oberflächenbereich des Gegenstands, wobei die Lage des ersten Markierungsfeldes (2) durch die Schablone (7) angezeigt wird,
c) Bereitstellen einer ersten Nachweissubstanz, die eine erste Nachweisnukleinsäure aufweist, die zumindest teilweise komplementär zu der ersten Markierungsnukleinsäure ist,
d) Aufbringen der ersten Nachweissubstanz auf das erste Markierungsfeld (2) unter Bedingungen, bei denen die erste Nachweisnukleinsäure spezifisch an die erste Markierungsnukleinsäure bindet, wobei durch die Bindung eine optische Eigenschaft der ersten Nachweisnukleinsäure verändert wird,
e) Messung der optischen Eigenschaft auf dem ersten Markierungsfeld (2) und
f) Identifizieren der Markierung anhand der optischen Eigenschaft auf dem ersten Markierungsfeld (2).

2. Verfahren nach Anspruch 1, wobei in der ersten Nachweissubstanz eine erste Nachweisnukleinsäure in einem Überschuss unspezifischer Nukleinsäure versteckt ist.

3. Verfahren nach Anspruch 1 oder 2, wobei der zu identifizierende Gegenstand (1) zumindest auf einem zweiten Referenzfeld (3) oberflächlich eine unspezifische Substanz enthält, die eine unspezifische oder keine Nukleinsäure aufweist.

4. Verfahren nach Anspruch 3, wobei die Schablone (7) auchdas zweite Referenzfeld (3) anzeigt.

5. Verfahren nach Anspruch 1 oder 2, wobei die Schablone (7) zumindest auf einem zweiten Referenzfeld (3) oberflächlich eine unspezifische Substanz enthält, die eine unspezifische oder keine Nukleinsäure aufweist.

6. Verfahren nach Anspruch 3, 4 oder 5, wobei die Nachweissubstanz auch auf das zweite Referenzfeld (3) aufgebracht wird, die optische Eigenschaft auf dem zweiten Referenzfeld (3) gemessen wird und die Markierung anhand des Verhältnisses der Messungen der optischen Eigenschaften auf dem ersten Markierungsfeld (2) und dem zweiten Referenzfeld (3) identifiziert wird.

7. Verfahren nach einem der vorhergehenden Ansprüche, wobei zusätzlich vor dem Aufbringen der Nachweissubstanz die optische Eigenschaft auf dem ersten Markierungsfeld (2) gemessen wird.

8. Verfahren nach Anspruch 7, wobei die Markierung anhand des Verhältnisses der Messungen der optischen Eigenschaften auf dem ersten Markierungsfeld (2) vor und nach Aufbringen der Nachweissubstanz identifiziert wird.

9. Verfahren nach einem der Ansprüche 3 bis 8, wobei das erste Markierungsfeld (2) und das zweite Referenzfeld (3) benachbart vorliegen.

10. Verfahren nach einem der vorherigen Ansprüche, wobei auf dem Gegenstand (1) optische Merkmale (6) im Umgebungsbereich des ersten Markierungsfeldes (2) und/oder des zweiten Referenzfeldes (3) aufgebracht sind.

11. Verfahren nach einem der vorherigen Ansprüche, wobei auf der Schablone (7) optische Merkmale (6) im Umgebungsbereich des ersten Markierungsfeldes (2) und/oder des zweiten Referenzfeldes (3) aufgebracht sind.

12. Verfahren nach einem der vorherigen Ansprüche, wobei die Schablone (7) mindestens eine Aussparung (4, 5) aufweist, durch welche die Nachweissubstanz aufgetragen werden kann.

13. Verfahren nach einem der vorherigen Ansprüche, wobei die Schablone (7) eine Mehrzahl von Aussparungen (4, 5) aufweist.

14. Verfahren nach einem der vorherigen Ansprüche, wobei die Schablone (7) eine Mehrzahl von ersten Markierungsfeldern (2) und/oder zweiten Referenzfeldern (3) anzeigt.

15. Verfahren nach einem der vorherigen Ansprüche, wobei die Schablone (7) sichtbare Markierungen (9) aufweist, um die Orientierung der Schablone (7) auf dem Gegenstand (1) zu erleichtern.

16. Verfahren nach einem der vorherigen Ansprüche, wobei der Gegenstand (1) sichtbare Markierungen (8) aufweist, um die Orientierung der Schablone (7) auf dem Gegenstand (1) zu erleichtern.

17. Verfahren nach einem der vorherigen Ansprüche, wobei die Schablone (7) eine dreidimensionale Geometrie aufweist, die zumindest partiell zu der dreidimensionalen Geometrie des Gegenstands (1) komplementär ist.

18. Verfahren nach einem der vorherigen Ansprüche, wobei die optische Eigenschaft die Intensität der Fluoreszenz ist.

19. Verfahren nach einem der vorherigen Ansprüche, wobei die erste Nachweisnukleinsäure ein Molecular Beacon ist.

20. Verfahren nach einem der vorherigen Ansprüche, wobei die Messung der optischen Eigenschaft mit einem Fluoreszenz-Handscanner erfolgt.

21. Verfahren nach einem der vorherigen Ansprüche, wobei das Identifizieren innerhalb weniger Minuten erfolgt.

22. Verfahren nach einem der vorherigen Ansprüche, wobei das Identifizieren innerhalb von zwei Minuten erfolgt.

23. Verfahren nach einem der vorherigen Ansprüche, wobei der Gegenstand (1) ein Dokument, ein Geldschein, eine Ware oder eine Verpackung ist.

24. Verfahren nach einem der vorherigen Ansprüche, wobei die Fläche eines ersten Markierungsfeldes (2) und/oder eines zweiten Referenzfeldes (3) höchstens wenige 1000 µm² beträgt.

25. Verfahren nach einem der vorherigen Ansprüche, wobei der Gegenstand (1) vor der Identifizierung dem freien Dokumenten-, Waren-, Güterverkehr ausgesetzt war.

26. Verfahren nach einem der vorherigen Ansprüche, wobei das erste Markierungsfeld (2) und/oder das zweite Referenzfeld (3) weiterhin optisch aktive Substanzen wie Fluorophore oder IR-aktive Stoffe aufweisen.

27. Verfahren nach Anspruch 26, wobei die optisch aktiven Substanzen für das Auge ohne Hilfsmittel unsichtbar sind.

28. Verfahren nach einem der vorherigen Ansprüche, wobei die erste Markierungsnukleinsäure eine synthetische Nukleinsäure ist.

29. Verfahren nach einem der vorherigen Ansprüche, wobei ein erstes Markierungsfeld (2) und/oder ein zweites Referenzfeld (3) die Form eines Balkens hat.

30. Kit zum Identifizieren eines markierten Gegenstands (1) zur Durchführung eines Verfahrens nach einem der vorhergehenden Ansprüche, wobei der Gegenstand eine oberflächlich aufgebrachten Markierungsubstanz an einem vorgegebenen Ort enthält, die eine erste Markierungsnukleinsäure mindestens teilweise bekannter Sequenz aufweist, mit
a) einer Schablone, wobei die Schablone auf den Gegenstand in definierter Weise so aufbringbar ist, dass sie nach Aufbringung den Ort der Markierung anzeigt, und
b) einer Flüssigkeit, die eine zur ersten Markierungsnukleinsäure zumindest teilweise komplementären Nachweisnukleinsäure enthält und die in einem Mittel zur volumendefinierten Abgabe der Flüssigkeit enthalten ist.

31. Kit, nach Anspruch 30 der weiterhin ein Detektionsgerät (Fluoreszenzdetektor) umfasst, das zum Nachweis einer Hybridisierung zwischen der ersten Markierungsnukleinsäure und ersten Nachweisnukleinsäure geeignet ist.

## Claims

1. Method for forgery-proof identification of a marking provided on an object (1), comprising the following steps:
a) providing an object (1) to be identified, the object comprising on its surface at least in a first marking field (2) a first marking substance for marking the object, the marking substance comprising a first nucleic acid having an at least partially known sequence,
b) applying a stencil (7) in a defined orientation on a given surface portion of the object, wherein the position of the first marking field (2) is indicated by the stencil (7),
c) providing a first detection substance which comprises a first detection nucleic acid which is at least partially complementary to the first marking nucleic acid,
d) applying the first detection substance to the first marking field (2) under conditions in which the first detection nucleic acid is specifically binding to the first marking nucleic acid, wherein an optical property of the first detection nucleic acid is changed by the binding,
e) measuring the optical property of the first marking field (2), and
f) identifying the marking on the basis of the optical property of the first marking field.

2. Method according to claim 1, wherein in the first detection substance a first detection nucleic acid is hidden in an excess of unspecific nucleic acid.

3. Method according to claim 1 or 2, wherein the object (1) to be identified comprises at least on a second reference field (3) on its surface an unspecific substance, which comprises an unspecific nucleic acid or no nucleic acid.

4. Method according to claim 3, wherein the stencil (7) is also indicating the second reference field (3).

5. Method according to claim 1 or 2, wherein the stencil (7) comprises at least on the second reference field (3) on the surface an unspecific substance which includes unspecific nucleic acids or no nucleic acid.

6. Method according to claim 3, 4 or 5, wherein the detection substance is also applied to the second reference field (3), the optical property is measured on the second reference field (3) and wherein the marking is identified on the basis of the ratio of the measurements of the optical properties of the first marking field (2) and of the second reference field (3).

7. Method according to any of the preceding claims, wherein prior to the application of the detection substance the optical property on the first marking field (2) is measured.

8. Method according to claim 7, wherein the marking is identified on the basis of the ratio of the measurements of the optical properties of the first marking field (2) before and after application of the detection substance.

9. Method according to any of the claims 3 to 8, wherein the marking field (2) and the second reference field (3) are adjacent to each other.

10. Method according to any of the preceding claims, wherein optical features (6) are provided on the object (1) in the area surrounding the first marking field (2) and/or the second reference field (3).

11. Method according to any of the preceding claims, wherein optical features (6) are provided on the stencil (7) in the area surrounding the first marking field (2) and/or the second reference field (3).

12. Method according to any of the preceding claims, wherein the stencil (7) comprises at least one opening (4, 5) through which detection substance can be applied.

13. Method according to any of the preceding claims, wherein the stencil (7) comprises a plurality of openings (4, 5).

14. Method according to any of the preceding claims, wherein the stencil (7) indicates a plurality of first marking fields (2) and/or second reference fields (3).

15. Method according to any of the preceding claims, wherein the stencil (7) includes visible markings (9) to facilitate the orientation of the stencil (7) on the object.

16. Method according to any of the preceding claims, wherein the object (1) comprises visible markings (8) in order to facilitate the orientation of the stencil (7) on the object (1).

17. Method according to any of the preceding claims, wherein the stencil (7) comprises a three dimensional geometry which is at least partially complementary to the three dimensional geometry of the object (1).

18. Method according to any of the preceding claims, wherein the optical property is the intensity of fluorescence.

19. Method according to any of the preceding claims, wherein the detection nucleic acid is a molecular beacon.

20. Method according to any of the preceding claims, wherein the measurement of the optical property is performed using a hand-held fluorescence scanner.

21. Method according to any of the preceding claims, wherein the identification is taking place within a few minutes.

22. Method according to any of the preceding claims, wherein the identification is taken place within two minutes.

23. Method according to any of the preceding claims, wherein the object (1) is a document, a banknote, an article or a package.

24. Method according to any of the preceding claims, wherein the area of a first marking field (2) and/or a second reference field (3) is at most a few 1000 µm².

25. Method according to any of the preceding claims, wherein the object (1) was, prior to the identification, exposed to free document exchange, goods traffic or commercial transportation.

26. Method according to any of the preceding claims, wherein the first marking field (2) and/or the second reference field (3) further comprise optically active substances such as fluorophores or IR-active substances.

27. Method according to claim 26, wherein the optically active substances are invisible for the eye without auxiliary means.

28. Method according to any of the preceding claims, wherein the first marking nucleic acid is a synthetic nucleic acid.

29. Method according to any of the preceding claims, wherein a first marking field (2) and/or a second reference field (3) have the shape of a bar.

30. Kit for identifying a marked object (1) for carrying out a method according to any of the preceding claims, wherein the object contains a marking substance applied to the surface in a predetermined position, which marking substance comprises a first detection nucleic acid of at least partially known sequence, comprising
a) a stencil, wherein the stencil can be applied to the object in a defined manner such that it indicates the position of the marking after its application, and
b) a liquid which comprises a detection nucleic acid at least partially complementary to the first marking nucleic acid, and which liquid is contained in means for dispensing defined volumes.

31. Kit according to claim 30, which further comprises a detection device (fluorescence detector), which is capable of detecting a hybridization between the first marking nucleic acid and the first detection nucleic acid.

## Revendications

1. Procédé pour l'identification infalsifiable d'un marquage prévu sur un objet (1) avec les étapes suivantes :
a) Mise à disposition d'un objet à identifier (1) qui contient une première substance de marquage en surface au moins dans un premier champ de marquage (2) pour le marquage de l'objet, première substance de marquage qui présente un premier acide nucléique de marquage de séquence au moins partiellement connue,
b) Application d'un gabarit (7) dans une orientation définie sur une zone de surface prédéfinie de l'objet, la position du premier champ de marquage (2) étant indiquée par le gabarit (7),
c) Mise à disposition d'une première substance de décèlement qui présente un premier acide nucléique de décèlement qui est au moins partiellement complémentaire du premier acide nucléique de marquage,
d) Application de la première substance de décèlement sur le premier champ de marquage (2) dans des conditions pour lesquelles le premier acide nucléique de décèlement se lie de manière spécifique au premier acide nucléique de marquage, une propriété optique du premier acide nucléique de décèlement étant modifiée par la liaison,
e) Mesure de la propriété optique sur le premier champ de marquage (2) et
f) Identification du marquage à l'aide de la propriété optique sur le premier champ de marquage (2).

2. Procédé selon la revendication 1, un premier acide nucléique de décèlement étant caché dans la première substance de décèlement dans un excès d'acide nucléique non spécifique.

3. Procédé selon la revendication 1 ou 2, l'objet à identifier (1) contenant en surface une substance non spécifique au moins sur un second champ de référence (2), substance non spécifique qui présente un acide nucléique non spécifique ou pas d'acide nucléique.

4. Procédé selon la revendication 3, le gabarit (7) indiquant également le second champ de référence (3).

5. Procédé selon la revendication 1 ou 2, le gabarit (7) contenant en surface une substance non spécifique au moins sur un second champ de référence (3), substance non spécifique qui présente un acide nucléique non spécifique ou pas d'acide nucléique.

6. Procédé selon la revendication 3, 4 ou 5, la substance de décèlement étant également appliquée sur le second champ de référence (3), la propriété optique étant mesurée sur le second champ de référence (3) et le marquage étant identifié à l'aide du rapport des mesures des propriétés optiques sur le premier champ de marquage (2) et sur le second champ de référence (3).

7. Procédé selon l'une des revendications précédentes, la propriété optique étant mesurée sur le premier champ de marquage (2) en plus avant l'application de la substance de décèlement.

8. Procédé selon la revendication 7, le marquage étant identifié à l'aide du rapport des mesures des propriétés optiques sur le premier champ de marquage (2) avant et après l'application de la substance de décèlement.

9. Procédé selon l'une des revendications 3 à 8, le premier champ de marquage (2) et le second champ de référence (3) étant voisins.

10. Procédé selon l'une des revendications précédentes, des caractéristiques optiques (6) étant appliquées sur l'objet (1) dans la zone environnante du premier champ de marquage (2) et/ou du second champ de référence (3).

11. Procédé selon l'une des revendications précédentes, des caractéristiques optiques (6) étant appliquées sur le gabarit (7) dans la zone environnante du premier champ de marquage (2) et/ou du second champ de référence (3).

12. Procédé selon l'une des revendications précédentes, le gabarit (7) présentant au moins un évidement (4, 5) à travers lequel la substance de décèlement peut être appliquée.

13. Procédé selon l'une des revendications précédentes, le gabarit (7) présentant une multitude d'évidements (4, 5).

14. Procédé selon l'une des revendications précédentes, le gabarit (7) indiquant une multitude de premiers champs de marquage (2) et/ou de seconds champs de référence (3).

15. Procédé selon l'une des revendications précédentes, le gabarit (7) présentant des marquages visibles (9) pour faciliter l'orientation du gabarit (7) sur l'objet (1).

16. Procédé selon l'une des revendications précédentes, l'objet (1) présentant des marquages visibles (8) pour faciliter l'orientation du gabarit (7) sur l'objet (1).

17. Procédé selon l'une des revendications précédentes, le gabarit (7) présentant une géométrie tridimensionnelle qui est au moins partiellement complémentaire de la géométrie tridimensionnelle de l'objet (1).

18. Procédé selon l'une des revendications précédentes, la propriété optique étant l'intensité de la fluorescence.

19. Procédé selon l'une des revendications précédentes, le premier acide nucléique de décèlement étant un phare moléculaire.

20. Procédé selon l'une des revendications précédentes, la mesure de la propriété optique étant effectuée avec un scanner à main à fluorescence.

21. Procédé selon l'une des revendications précédentes, l'identification se faisant en quelques minutes.

22. Procédé selon l'une des revendications précédentes, l'identification se faisant en deux minutes.

23. Procédé selon l'une des revendications précédentes, l'objet (1) étant un document, un billet, une marchandise ou un emballage.

24. Procédé selon l'une des revendications précédentes, la surface d'un premier champ de marquage (2) et/ou d'un second champ de référence (3) étant de quelques 1000 µm² au maximum.

25. Procédé selon l'une des revendications précédentes, l'objet (1) étant exposé, avant l'identification, à la libre circulation des documents, des produits, des marchandises.

26. Procédé selon l'une des revendications précédentes, le premier champ de marquage (2) et/ou le second champ de référence (3) présentant de plus des substances actives optiquement comme les fluorophores ou les matières actives infrarouges.

27. Procédé selon la revendication 26, les substances actives optiquement étant invisibles à l'oeil sans moyens auxiliaires.

28. Procédé selon l'une des revendications précédentes, le premier acide nucléique de marquage étant un acide nucléique synthétique.

29. Procédé selon l'une des revendications précédentes, un premier champ de marquage (2) et/ou un second champ de référence (3) ayant la forme d'une barre.

30. Kit pour l'identification d'un objet marqué (1) pour exécuter un procédé selon l'une des revendications précédentes, l'objet contenant une substance de marquage appliquée en surface à un endroit prédéfini qui présente un premier acide nucléique de marquage de séquence au moins partiellement connue avec
a) un gabarit, le gabarit pouvant être appliqué sur l'objet de manière définie de telle manière qu'il indique, après application, l'endroit du marquage et
b) un liquide qui contient un acide nucléique de décèlement au moins partiellement complémentaire du premier acide nucléique de marquage et qui est contenu dans un moyen pour délivrer un volume défini de liquide.

31. Kit selon la revendication 30 qui comprend de plus un appareil de détection (détecteur par fluorescence) qui est approprié pour déceler une hybridation entre le premier acide nucléique de marquage et le premier acide nucléique de décèlement.
